# EUROPEAN PATENT SPECIFICATION

(11) **EP 2 080 800 B1**
(45) Date of publication and mention of the grant of the patent: **14.03.2012**
(21) Application number: 09000733.7
(22) Date of filing: 20.01.2009
(51) Int. Cl.: C12N 5/00

(54) **Production method for a porous cell scaffold.**
Herstellungmethode für ein poröses Zellengerüst.
Procédé de production d'une structure poreuse cellulaire.

(30) Priority: 21.01.2008 JP 2008010882; 16.12.2008 JP 2008320040
(43) Date of publication of application: 22.07.2009
(73) Proprietor: GC Corporation, Itabashi-ku Tokyo 174-8585 (JP)
(72) Inventor: Yamanaka, Katsuyuki, Tokyo 174-8585 (JP); Suda, Youko, Tokyo 174-8585 (JP); Yamamoto, Katsushi, Tokyo 174-8585 (JP); Sakai, Yuhiro, Tokyo 174-8585 (JP); Kaneko, Tadashi, Tokyo 174-8585 (JP)
(74) Representative: Müller-Boré & Partner Patentanwälte

(56) References cited:
- WO-A-2005/005607
- LI Y ET AL: "Effects of filtration seeding on cell density, spatial distribution, and proliferation in nonwoven fibrous matrices" BIOTECHNOLOGY PROGRESS 2001 AMERICAN CHEMICAL SOCIETY US, vol. 17, no. 5, 2001, pages 935-944, XP002522161
- XIE J ET AL: "New technique of seeding chondrocytes into microporous poly(L-lactide-co- [epsilon]-caprolactone) sponge by cyclic compression force-induced suction" TISSUE ENGINEERING JULY 2006 MARY ANN LIEBERT INC. US, vol. 12, no. 7, July 2006 (2006-07), pages 1811-1820, XP002522162
- HOLLISTER SCOTT J: "Porous scaffold design for tissue engineering." NATURE MATERIALS JUL 2005, vol. 4, no. 7, July 2005 (2005-07), pages 518-524, XP002522163 ISSN: 1476-1122

## Description

The present invention relates to a production method for a porous cell scaffold having a thickness of 2 mm or more and having an internal small hole structure wholly seeded with cells.

A scaffold having a porous structure which acts as a substrate seeded with cells is generally used in a cell technology field. Cells seeded in a scaffold formed to have a required shape are transplanted into a living body for every scaffold and used. It is desired to almost uniformly seed cells in the scaffold. Since the scaffold has a porous three-dimensional structure having a small hole structure, it is hard to fill a culture medium with cells being suspended therein (it will be called simply a cell suspension liquid below) to a center part of the scaffold. For example, it is remarkably hard to seed cells in a whole scaffold having a thickness of 2 mm or more.

Unexamined Japanese Patent Application Laid-Open No. 2005-160596, for example discloses a method including the steps of mixing micro moldings in a cell suspension liquid, culturing the mixture until cell aggregated masses are formed in the micro moldings so as to form complex bodies of the micro moldings and cells into a two-dimensional or three-dimensional cell assembly. However, this method needs many steps of separately preparing micro moldings, culturing until cell aggregated masses are formed in the micro moldings so as to form the complex bodies of the micro moldings and cells, and making the complex bodies of the micro moldings and cells into a two-dimensional or three-dimensional cell assembly. In addition, this method involves another problem that it is hard to obtain a scaffold having a desired external shape.

In order to solve the problems, for example, Unexamined Japanese Patent Application Laid-Open No. 2006-508717 proposes in claim 8 thereof a method including the steps of sinking a scaffold in a cell suspension liquid, holding the scaffold under a state of a lower pressure than an atmospheric pressure or vacuum, and permeating the cell suspension liquid into the scaffold, in Claim 8. However, since it is hard to permeate the cell suspension liquid into the whole of the scaffold by only utilizing a negative pressure, a path for permeating the cell suspension liquid is formed with a laser light or the like in this method. Further, it is not preferable for cells to greatly change a pressure by utilizing a negative pressure. Further, since the amount of cells is specified by the concentration in the suspension liquid, there is a problem that it is hard to attach an actual amount of cells contained in a scaffold.

Further, Unexamined Japanese Patent Application Laid-Open No. 2007-181459 discloses a method including the steps of taking a cell suspension liquid on a scaffold and entering cells into the scaffold by using centrifugal force. However, also in this method, since it is hard to seed cells in a whole scaffold and high force is applied to the cells, it is not preferable.

Y. Li et al., Biotechnology Progress 2001, vol. 17, no. 5, 2001, 935-944, disclose a study on the effects of filtration seeding on cell density, spatial distribution and proliferation in nonwoven fibrous matrices.

J. Xie et al., Tissue Engineering, vol. 12, no. 7, 2006, 1811-1820, disclose a technique of seeding chondrocytes into microporous poly(L-lactide-co-ε-caprolactone) sponge by cyclic compression force-induced suction.

S. J. Hollister, Nature Materials, vol. 4, no. 7, 2005, 518-524, discloses a review on porous scaffold design for tissue engineering.

The present invention is to solve the above problems, and an objective of the present invention is to provide a production method for a porous cell scaffold having a thickness of 2 mm or more, in which cells can be easily seeded in a whole small hole structure of a scaffold, high force is not applied to the cells, and an amount of cells in the scaffold can be almost accurately attached.

Present inventors carried out earnest works to solve the above-described problems and, as a result, they found out the followings to complete the present invention. A guiding solution is filled in a whole continuous small hole structure of a sheet-shaped or block-shaped scaffold having the continuous small hole structure with hole diameters of 5 to 3200 µm and an average hole diameter of 50 to 1500 µm and having a thickness of 2 mm or more, a culture medium with cells being suspended (a cell suspension liquid) is thereafter supplied to an upper side of the scaffold, the guiding solution is sucked from a lower side of the scaffold by means of a water absorber contacting the lower side of the scaffold, and enters thereby the culture medium with cells being suspended into the whole small hole structure of the scaffold. Thus, negative pressure is generated between the guiding solution sucked from the lower side of the scaffold and the cell suspension liquid supplied to the upper side of the scaffold. Consequently, the supplied cell suspension liquid follows the flow of the sucked guiding solution, and is filled in the whole small hole structure of the scaffold by replacing of the guiding solution with the cell suspension liquid. Therefore, the cells can be accurately seeded in the whole small hole structure of the scaffold.

That is to say, an aspect of the present invention is a production method of a porous cell scaffold including the steps of filling a guiding solution in a whole continuous small hole structure of a sheet-shaped or block-shaped scaffold having the continuous small hole structure with hole diameters of 5 to 3200µm and an average hole diameter of 50 to 1500µm, and having a thickness of 2 mm or more, supplying thereafter a culture medium with cells being suspended to an upper side of the scaffold, sucking the guiding solution from a lower side of the scaffolld by means of a water absorber contacting the lower side of the scaffold, and entering thereby the culture medium with cells being suspended into the whole small hole structure of the scaffold, wherein the water absorber is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing polymer materials.

Further, in the production method of the porous cell scaffold according to the present invention, if kinematic viscosity of the guiding solution at a time of use is 50 to 450% of kinematic viscosity of the culture medium at a time of use, there is no great difference in kinematic viscosity between the guiding solution previously contained in the scaffold and the culture medium which replaces the guiding solution.

Thus, the culture medium can replace the guiding solution smoothly and accurately, so it is preferable. As for the guiding solution having such the kinematic viscosity, water, physiological saline, a buffer solution, body fluid, and a culture medium are preferable because those are easily sucked. Further, if a means for sucking the guiding solution from the lower side of the scaffold is a water absorber which contacts the lower side of the scaffold, the guiding solution can be easily sucked without using a complicated suction device, so it is preferable. According to the present invention the water absorber is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing polymer materials, because these absorbers can be gotten easily.

Further, according to a preferred embodiment a porous cell scaffold having high cell density and being more proper for a medical treatment can be easily produced by supplying a new culture medium from upside to the porous cell scaffold produded by the above-mentioned steps, where the culture medium with cells being suspended is entered at every predetermined period of time passing, removing the culture medium from the lower side of the scaffold by means of a water absorber contacting the lower side of the scaffold, repeating such change of the culture medium, and producing thereby a porous cell scaffold containing cultured cells, wherein the water absorber contacting the lower side of the scaffold is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing polymer materials since a means for removing the culture medium from the lower side of the scaffold is a water absorber contacting the lower side of the scaffold, the culture medium can be removed easily, The above-mentioned absorbers can be gotten easily and used simply.

By the above-described method, negative pressure is generated between the guiding solution sucked from the lower side of the scaffold and the cell suspension liquid supplied to the upper side of the scaffold. As a result, the supplied cell suspension liquid follows the flow of the sucked guiding solution, and is filled in the whole small hole structure of the scaffold by replacing of the guiding solution with the cell suspension liquid. Thus, the culture medium with cells being suspended can be accurately entered into the whole small hole structure of the scaffold. Consequently, such a porous cell scaffold having a thickness of 2 mm or more and having a small hole structure in which cells are wholly seeded, as is hardly produced by conventional techniques, can be easily produced. Further, since high force is not applied to the cell scaffold at the time of production, a large stress is not applied to the cells. Furthermore, the amount of cells seeded in the whole small hole structure of the scaffold can be gasped easily from the amount of supplied cell suspension liquid.

Further, the porous cell scaffold produced by the present invention method to have a thickness of 2 mm or more and have a small hole structure in which cells are wholly seeded, has a sufficient thickness so that the porous cell scaffold can be used in a remarkably wide application, and can be widely used even in a field in which a conventional scaffold is hardly used.

Further, in the production method of the porous cell scaffold according to the present invention, if kinematic viscosity of the guiding solution at a time of its use is 50 to 450% of kinematic viscosity of the culture medium at a time of its use, there is no great difference in kinematic viscosity between the guiding solution previously contained in the scaffold and the culture medium which replaces the guiding solution. Thus, the culture medium can replace the guiding solution smoothly and accurately. As for the guiding solution having such the kinematic viscosity, water, physiological saline, a buffer solution, body fluid, or a culture medium can be sucked easily. Further, if a means for sucking the guiding solution from the lower side of the scaffold is a water absorber which contacts the lower side of the scaffold, the guiding solution can be easily sucked without using a complicated suction device, and the guiding solution is not sucked excessively, so a large stress is not applied to the cells. Furthermore, since the water absorber is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing high polymer materials, these absorbers can be gotten easily and used simply.

Further, a porous cell scaffold having high cell density and being more proper for a medical treatment can be easily produced, if cells are cultured by supplying a new culture medium from upside to the porous cell scaffold, where the culture medium with cells being suspended is already entered, at every predetermined period of time passing, removing the culture medium from the lower side of the scaffold, and repeating such change of the culture medium, according to the production method of the porous cell scaffold in the present invention as mentioned above. Further, since means for removing the culture medium from the lower side of the scaffold is a water absorber contacting the lower side of the scaffold, the culture medium can be removed easily. Furthermore, since the water absorber contacting the lower side of the scaffold is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing polymer materials, these absorbers can be gotten easily and used simply.

A scaffold obtained by the method of the present invention is a sheet-shaped or block-shaped scaffold having a thickness of 2 mm or more, and having a continuous small hole structure with hole diameters of 5 to 3200µm and an average hole diameter of 50 to 1500µm. A conventionally used material can be used for such a scaffold. For example, the material is at least one kind selected from polyglycolic acid, polylactic acid, a lactic acid/glycolic acid copolymer, poly-ε-caprolactone, a lactic acid/ε-caprolactone copolymer, polyamino acid, polyorthoester, polymalic acid, and a copolymer of those, at least one kind selected from collagen, chitosan, hyaluronic acid, alginate, and a complex of those, or at least one kind selected from calcium phosphate, β-TCP, α-TCP, hydroxyapatite, carbonate group-containing hydroxyapatite, and a complex of those. These materials can be used by combining them.

As for a guiding solution to be filled in a whole continuous small hole structure of the scaffold, if kinematic viscosity of the guiding solution at a time of use is 50 to 450% of kinematic viscosity of the culture medium at a time of use, there is no great difference in kinematic viscosity between the guiding solution previously contained in the scaffold and the culture medium which replaces the guiding solution. Thus, the culture medium can replace the guiding solution smoothly and accurately. Water, physiological saline, a buffer solution, body fluid, and a culture medium can be preferably used.

The cells suspended in the culture medium are not restricted especially. However, for example, the cells include epidermal cells, keratinocyte cells, fat cells, hepatocytes, neurons, nerve cells neuroglias, astroglias, epitheliocytes, breast epidermal cells, endothelial cells, mesenchymal cells, dermal fibroblasts, mesothelial cells, osteo blast cells, smooth muscle cells, rhabdomyoblast, ligament fibroblasts, tendon fibroblast, chondrocytes, marrow cells, osteoblasts, dental pulp cells, periodontal cells, pulp cells, somatic stem cells, ES cells or the like.

The method for sucking the guiding solution from the lower side of the scaffold is such that the sucking means is a water absorber contacting the lower side of the scaffold, whereby the guiding solution can be easily sucked without using a complicated suction device, and the guiding solution is not sucked excessively, so that a large stress is not applied to the cells. The water absorber used at that time is a material which can properly suck the guiding solution from the scaffold and is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing polymer materials, because these absorbers can be gotten easily and used simply.

In order to actually produce a porous cell scaffold by the production method according to the present invention, a sheet-shaped or block-shaped scaffold having a thickness of 2 mm or more and having a continuous small hole structure with hole diameters of 5 to 3200µm and an average hole diameter of 50 to 1500µm is prepared. As for the scaffold, a bioabsorbable polymer material and a calcium phosphate material, which are conventionally used, can be used. A scaffold not having a continuous small hole structure cannot suck the guiding solution, and cannot enter the culture medium with cells being suspended into the whole small hole structure of the scaffold. Thus, the scaffold not having the continuous small hole structure cannot be used for the production method according to the present invention.

As for a method for producing a scaffold having a continuous small hole structure, when the bioabsorbable polymer material is used, the method includes the steps of dissolving the bioabsorbable polymer material with a solvent, mixing it with sodium chloride having a predetermined particle diameter, freeze-drying the mixture, pulverizing it, desalting it so as to acquire a powdered material, and molding the powdered material by compressing and heating. When the calcium phosphate material is used, methods disclosed in Unexamined Japanese Patent Applications Laid-Open Nos. 5-237178, 2002-58688, 2002-17846, and 2004-59344 can be used.

Next, it is necessary to fill a guiding solution in the whole continuous small hole structure of the scaffold. The guiding solution is a solution used to be replaced with the culture medium with cells being suspended and, of course, it does not contain cells. Thus, the guiding solution can be forcibly entered into the scaffold by applying force to it. For example, the scaffold may be dipped in the guiding solution and placed under a reduced-pressure state, so that the guiding solution is infiltrated into the scaffold.

After the guiding solution is thus filled in the whole continuous small hole structure, the culture medium with cells being suspended supplied to an upper side of the scaffold, and the guiding solution is sucked from a lower side of the scaffold, whereby the culture medium with cells being suspended enters into the whole small hole structure of the scaffold. Such supply of the culture medium with cells being suspended to the upper side of the scaffold and suction of the guiding solution from the lower side can be carried out almost simultaneously. Further, the suction of the guiding solution from the lower side of the scaffold is first started, and then supply of the culture medium with cells being suspended to the upper side of the scaffold can be started quickly before a lot of the guiding solution is sucked. Further, if a frame is fitted to the side face of the scaffold, it can be prevented to flow the culture medium with cells being suspended to the water absorber along the outer side of the scaffold.

Next, as for a method for culturing the cells entered into the porous cell scaffold, whenever a predetermined period of time passes, a new culture medium is supplied from an upside to the porous cell scaffold, where the culture medium with cells being suspended is entered, by sucking the guiding solution from the lower side of the scaffold so that the culture medium with cells being suspended replaces the guiding solution, the culture medium is removed from the lower side of the scaffold, and such change of the culture medium is repeated, as described in detail above.

As for a method for removing the culture medium from the lower side of the scaffold, a water absorber is made in contact with the lower side of the scaffold, such that the culture medium can be changed easily by only replacing the water absorber. Further, the water absorber used at that time is a material which can remove the culture medium from the scaffold is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing polymer materials, because these absorbers can be gotten easily and used simply.

The porous cell scaffold produced according to the present invention as explained above has a thickness of 2 mm or more, and cells are seeded in a whole small hole structure and also cultured. Thus, the porous cell scaffold can be used for a remarkably wide application and, especially, contributes greatly to a medical field.

### [Examples]

The present invention will be described in detail below with examples, but the present invention is not limited to these examples.

### <Preparation of transplant cells>

### Transplant cells 1: Mesenchymal stem cells isolated from bone marrow of a human ilium

Cells isolated from bone marrow of a human ilium were suspended with 10% FBS in DMEM(Dullbecco's Modified Eagle's Medium), and seeded on a culturing dish at a density of nucleated cells of 1.8×10⁹ cells/cm². The cells were cultured under the existence of 5% carbon dioxide at 37°C. The culture medium was changed on the 3rd day so as to remove non-adherent cells (hematopoietic cells). Thereafter, the culture medium was changed once every three days. bFGF was added to the culture medium at the ratio of 3 ng/ml from the 5th day. The cells were proliferated to become almost dense on about the 10th day. These culture dishes were incubated for 5 minutes with trypsin (0.05%) and EDTA (0.2mM) and the cells were collected. The number of cells was measured by Coulter counter (Z1 Single, produced by Beckman Coulter Corporation), and the cells were seeded on the second culturing dish at the density of 5×10³ cells/cm².

The operation from the culturing of the cells under the existence of 5% carbon dioxide at 37°C to the incubating of the culturing dish for 5 minutes so as to collect the cells was repeated again, and the third generation cells acquired from the second culturing dish were used.

### Transplant cells 2: Mesenchymal stem cells isolated from femur and tibia of a rabbit

A femur and a tibia of a 6 week-age rabbit were removed except for muscles, and the like, and both ends of the femur and tibia were cut. The inside of a marrow was washed with a DMEM culture medium (10% FBS, penicillin of 32 units/ml, and streptomycin of 50 µg/ml). The washing liquid was collected and suspended well, and then 300 g of the washing liquid was subjected to centrifugal separation so as to separate cells. Approximately 7×10⁷ nucleated cells were acquired from the bone marrow. Cells isolated from the bone marrow were seeded on a culturing flask at the density of nucleated cells of 5×10⁵ cells/cm², and were cultured under an existence of 5% carbon dioxide at 37°C. The culturing liquid was changed on the 3rd day, and thereafter changed once every three days. bFGF was added to the culture medium at a ratio of 3 ng/ml from the 5th day. The cells were proliferated to become almost dense on about the 10th day. These culture dishes were incubated for 5 minutes by trypsin (0.05%) and EDTA (0.2mM) and the cells were collected. The number of cells was measured by Coulter counter (Z1 Single, produced by Beckman Coulter Corporation), and the cells were seeded on the second culturing dish at the density of 5×10³ cells/cm².

The operation from the culturing of the cells under the existence of 5% carbon dioxide at 37°C to the incubating of the culturing dish for 5 minutes so as to collect the cells was repeated again, and the third generation cells acquired from the second culturing dish were used.

### Transplant cells 3: Mesenchymal stem cells isolated from femur and tibia of a rat

A femur and tibia of a 4 week-age rat were removed except for muscles, ligaments and the like, and both ends of the femur and tibia were cut. The inside of a marrow was washed with a DMEM culture medium (having 10% FBS, penicillin of 32 units/ml, streptomycin of 50 µg/ml). The washing liquid was collected and suspended well, and then 300 g of the washing liquid was subjected to centrifugal separation so as to separate cells. Approximately 7×10⁷ nucleated cells were obtained from the bone marrow. Cells isolated from the bone marrow were seeded on a culturing flask at the density of nucleated cells of 5×10⁵ cells/cm², and were cultured under an existence of 5% carbon dioxide at 37°C. The culturing liquid was changed on the 3rd day, and then changed once every three days. bFGF was added to the culture medium at a ratio of 3 ng/ml from the 5th day. The cells were proliferated to become almost dense on about the 10th day. These culture dishes were incubated for 5 minutes by trypsin (0.05%) and EDTA (0.2mM) and the cells were collected. The number of cells was measured by Coulter counter (Z1 Single, produced by Beckman Coulter Corporation), and the cells were seeded on the second culturing dish at the density of 5×10³ cells/cm².

The operation from culturing of the cells under the existence of 5% carbon dioxide at 37°C to incubating of the culturing dish for 5 minutes so as to collect the cells was repeated again, and third generation cells acquired from the second culturing dish were used.

### <Preparation of a scaffold>

### Scaffold 1: Block of a polylactic acid / glycolic acid copolymer (PLGA)

A block-shaped porous body was produced by dissolving a DL-lactic acid / glycolic acid copolymer having a molecular weight of 250,000 with dioxane, mixing the solution with sodium chloride having a particle diameter of about 500µm, freeze-drying the mixture, pulverizing it so as to acquire a powder, and desalting the powder to acquire a powdered material, and molding the powdered material by compressing and heating. The produced block-shaped porous body had a continuous small hole structure with hole diameters of 18 to 310µm and an average hole diameter of 100µm, had a porosity of about 80%, a diameter of 9 mm, and a thickness of 2 mm and was made of a bioabsorbable synthetic polymer.

### Scaffold 2: Block of a polylactic acid / glycolic acid copolymer (PLGA) and collagen

A block-shaped porous body was produced by dipping the scaffold 1 in a 0.01% collagen solution, placing it under a reduced-pressure state, infiltrating the collagen solution into the scaffold 1, and drying it at a room temperature. The produced porous body was coated with a one layered collagen on a surface thereof, had a continuous small hole structure with hole diameters of 15 to 300µm and an average hole diameter of 170µm, and had a porosity of about 75%.

### Scaffold 3: β calcium phosphate (β-TCP) porous body

A calcium phosphate-based porous body having a diameter of 9 mm and a thickness of 2.2 mm was produced by a method disclosed in Unexamined Japanese Patent Application Laid-Open No. 5-237178 (or 2002-58688). The produced porous body had a continuous small hole structure with hole diameters of 70 to 420µm and an average hole diameter of 200µm, and had a porosity of about 75%.

### Scaffold 4: P calcium phosphate (β-TCP) porous body and collagen

A porous body was produced by dipping the scaffold 3 in a 0.01% collagen solution, placing it under a reduced-pressure state, infiltrating the collagen solution into the scaffold 3, and drying it at a room temperature. The produced porous body was coated with a one layered collagen on a surface thereof, had a continuous small hole structure with hole diameters of 55 to 400µm and an average hole diameter of 190µm, and had a porosity of about 72%.

### Scaffold 5: Hydroxyapatite (HAP) porous body

A porous body made of a calcium phosphate-based sintered body and having a diameter of 9 mm and a thickness of 2,2 mm, was produced by a method disclosed in Unexamined Japanese Patent Application Laid-Open No. 2002-17846 (or 2004-59344). The produced porous body had a continuous small hole structure with hole diameters of 23 to 300µm and an average hole diameter of 150µm, and had a porosity of about 75%.

### Scaffold 6: Hydroxyapatite (HAP) porous body and collagen

A porous body was produced by dipping the scaffold 5 in a 0.01% collagen solution, placing it under a reduced-pressure state, infiltrating the collagen solution into the scaffold 5, and drying it at a room temperature. The produced porous body was coated with a one layered collagen on a surface thereof, had a continuous small hole structure with hole diameters of 18 to 290µm and an average hole diameter of 135µm, and had a porosity of about 72%.

### Scaffold 7: Block of a polylactic acid / glycolic acid copolymer (PLGA)

A block-shaped porous body was produced by dissolving a DL-lactic acid / glycolic acid copolymer having a molecular weight of 250,000 with dioxane, mixing the solution with sodium chloride having a particle diameter of about 500µm, freeze-drying the mixture, pulverizing it so as to acquire a powder, desalting the powder to acquire a powdered material, and molding the powdered material by compressing and heating. The produced porous body had a continuous small hole structure with hole diameters of 546 to 1446µm and an average hole diameter of 910µm, had a porosity of about 92%, a diameter of 9 mm, and a thickness of 2 mm, and was made of a bioabsorbable synthetic polymer.

### Scaffold 8: Block of a hydroxyapatite (HAP) -dispersed polylactic acid / glycolic acid copolymer (PLGA)

A block-shaped porous body was produced by dissolving a DL-lactic acid / glycolic acid copolymer having a molecular weight of 250,000 with dioxane, mixing the solution with sodium chloride having a particle diameter of about 500µm, freeze-drying the mixture, pulverizing it so as to acquire a powder, desalting the powder to acquire a HAP-dispersed PLGA powdered material, and molding the powdered material by compressing and heating. The produced porous body had a continuous small hole structure with hole diameters of 18 to 310µm and an average hole diameter of 180µm, had a porosity of about 80%, a diameter of 9 mm and a thickness of 2 mm and was made of a bioabsorbable synthetic polymer.

### Scaffold 9: Block of a polylactic acid / glycolic acid copolymer (PLGA)

A block-shaped porous body was produced by dissolving a DL-lactic acid / glycolic acid copolymer having a molecular weight of 250,000 with dioxane, mixing the solution with sodium chloride having a particle diameter of about 500µm and HAP having a particle diameter of about 20µm, freeze-drying the mixture, pulverizing it so as to acquire a powder, desalting the powder to acquire a powdered material, and molding the powdered material by compressing and heating. The produced porous body had a continuous small hole structure with hole diameters of 714 to 3118µm and an average hole diameter of 1340µm, and had a porosity of about 90%, a diameter of 9 mm and a thickness of 4 mm and was made of a bioabsorbable synthetic polymer.

A cell transplantation treatment material was produced by dipping each of the support bodies prepared as mentioned above in a DMEM culture medium which is a guiding solution, taking it into a sterilized desiccator, reducing a pressure in the desiccator by a vacuum pump so as to make a pressure-reduction state, infiltrating the guiding solution inside the scaffold, transferring it onto a water absorber which was a sterilized filter paper (a product name: 26-WA, produced by ADVANTEC Corporation) made from a paper having a thickness of about 0.7 mm, suspending and supplying the transplant cells prepared as mentioned above into a culture medium as mentioned below so as to seed the cells, and culturing the cells under each condition. In addition, the scaffold has an approximately white color, and the seeding state of each transplant cell was confirmed by coloring of each transplant cell in the scaffold with a succinic acid dehydrogenase.

### <Example 1>

Transplant cells 1 were suspended with an osteogenic differentiation medium (αMEM, glucose of 1.0 mg/ml, 10% FBS, 10⁻⁷M dexamethasone, ascorbate-2-phosphate of 50µg/ml, β- glycerophosphate of 10mM) at the density of 2×10⁶ cells/cm². 0.5 ml of the suspended transplant cells 1 were supplied and seeded in the scaffold 1, and kept to stand for 2 hours. Then, the scaffold 1 together with the differentiation medium was transferred to a centrifugal tube containing a differentiation medium of 50ml, and rotated and cultured by a rotating incubator at 37°C for two days. When the scaffold 1 was cut to observe the inside thereof, the transplant cells 1 were seeded in an approximately whole small hole structure of the scaffold 1 having a thickness of 2.2 mm.

### <Example 2>

Transplant cells 1 were suspended with an osteogenic differentiation medium (αMEM, glucose of 1.0 mg/ml, 10% FBS, 10⁻⁷M dexamethasone, ascorbate-2-phosphate of 50µg/ml, β- glycerophosphate of 10mM) at the density of 2×10⁶ cells/cm². 0.5 ml of the suspended transplant cells 1 were supplied and seeded in the scaffold 1. The scaffold 1 together with the differentiation medium was entered into a centrifugal tube containing a differentiation medium of 50ml, and rotated and cultured at 37°C for four weeks, while the differentiation medium was changed once every three days by the following method. The differentiation medium in the scaffold 1 was changed by taking out the scaffold 1 from the differentiation medium once every three days, placing the scaffold 1 on a water absorber which was a filter paper (a product name: 26-WA, produced by ADVANTEC Corporation) made from a paper having a thickness of about 0.7 mm, and supplying the osteogenic differentiation medium from the upside of the scaffold 1 so as to change the differentiation medium in the scaffold 1. When the scaffold 1 was cut to observe the inside of it, the transplant cells 1 were seeded in an approximately whole small hole structure of the scaffold 1, like Example 1.

### <Example 3>

Transplant cells 1 were suspended with a chondrogenic differentiation medium (αMEM, glucose of 4.5 mg/ml, 10⁻⁷M dexamethasone, ascorbate-2-phosphate of 50µg/ml, TGF-β3 of 10 ng/ml, insulin of 6.25µg/ml, transferrin of 6.25µg/ml, selenic acid of 6.25µg/ml, linoleic acid of 5.33µg/ml, bovine serum albumin of 1.25 mg/ml) at the density of 2×10⁶ cells/cm². 0.5 ml of the transplant cells 1 were supplied and seeded in the scaffold 1. The scaffold 1 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 1 according to Example 3 was cut to observe the inside of it, the transplant cells 1 were seeded in an approximately whole small hole structure of the scaffold 1.

### <Example 4>

Transplant cells 1 were suspended with a adipogenic differentiation medium (DMEM, glucose of 4.5 mg/ml, 10% FBS, insulin of 10µg/ml, indomethacin of 0.2mM, 10⁻⁶M dexamethasone, 3-isobutyl-1-methylxanthine of 0.5mM) at the density of 2×10⁶ cells/cm². 0.5 ml of the transplant cells 1 were supplied and seeded in the scaffold 1. The scaffold 1 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 1 according to Example 4 was cut to observe the inside of it, the transplant cells 1 were seeded in an approximately whole small hole structure of the scaffold 1.

### <Example 5>

Transplant cells 2 were suspended with a chondrogenic differentiation medium (αMEM, glucose of 4.5 mg/ml, 10⁻⁷M dexamethasone, ascorbate-2-phosphate of 50µg/ml, TGF-β3 of 10 ng/ml, insulin of 6.25µg/ml, transferrin of 6.25µg/ml, selenic acid of 6.25µg/ml, linoleic acid of 5.33µg/ml, bovine serum albumin of 1.25 mg/ml) at the density of 2×10⁶ cells/cm². 0.5 ml of the transplant cells 2 were supplied and seeded in the scaffold 2. The scaffold 2 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 2 according to Example 5 was cut to observe the inside of it, the transplant cells 2 were seeded in an approximately whole small hole structure of the scaffold 2.

### <Example 6>

Transplant cells 3 were suspended with an osteogenic differentiation medium (αMEM, glucose of 1.0 mg/ml, 10% FBS, 10⁻⁷M dexamethasone, ascorbate-2-phosphate of 50µg/ml, β- glycerophosphate of 10mM) at the density of 2×10⁶ cells/cm². 0.5 ml of the transplant cells 3 were supplied and seeded in the scaffold 1. The scaffold 1 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 1 according to Example 6 was cut to observe the inside of it, the transplant cells 3 were seeded in an approximately whole small hole structure of the scaffold 1.

### <Example 7>

Transplant cells 3 were suspended with an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM) at the density of 2×10⁶ cells/cm². 0.5 ml of the transplant cells 3 were supplied and seeded in the scaffold 2. The scaffold 2 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 2 according to Example 7 was cut to observe the inside of it, the transplant cells 3 were seeded in an approximately whole small hole structure of the scaffold 2.

### <Example 8>

Transplant cells 1 were suspended with an osteogenic differentiation medium (αMEM, glucose of 1.0 mg/ml, 10% FBS, 10⁻⁷m dexamethasone, ascorbate-2-phosphate of 50µg/ml, β-glycerophosphate of 10mM) at the density of 2×10⁶ cells/cm². 0.5 ml of the transplant cells 1 were supplied and seeded in the scaffold 3. The scaffold 3 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 3 according to Example 8 was cut to observe the inside of it, the transplant cells 1 were seeded in an approximately whole small hole structure of the scaffold 3.

### <Example 9>

Transplant cells 2 were suspended with an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM) at the density of 2×10⁶ cells/cm². 0.5 ml of the transplant cells 2 were supplied and seeded in the scaffold 4. The scaffold 4 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 4 according to Example 9 was cut to observe the inside of it, the transplant cells 2 were seeded in an approximately whole small hole structure of the scaffold 4.

### <Example 10>

Transplant cells 3 were suspended with an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM) at the density of 2×10⁶ cells/cm². 0.5 ml of the transplant cells 3 were supplied and seeded in the scaffold 5. The scaffold 5 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 5 according to Example 10 was cut to observe the inside of it, the transplant cells 3 were seeded in an approximately whole small hole structure of the scaffold 5.

### <Example 11>

Transplant cells 3 were suspended with an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM) at the density of 2×10⁶ cells/cm². 0.5 ml of the transplant cells 3 were supplied and seeded in the scaffold 6. The scaffold 6 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 6 according to Example 11 was cut to observe the inside of it, the transplant cells 3 were seeded in an approximately whole small hole structure of the scaffold 6.

### <Example 12>

Transplant cells 1 were suspended with an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM) at the density of 2×10⁶ cells/cm². 0.5 ml of the transplant cells 1 were supplied and seeded in the scaffold 5. The scaffold 5 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 5 according to Example 12 was cut to observe the inside of it, the transplant cells 1 were seeded in an approximately whole small hole structure of the scaffold 5.

### <Example 13>

Transplant cells 2 were suspended with a chondrogenic differentiation medium (αMEM, glucose of 4.5 mg/ml, 10⁻⁷M dexamethasone, ascorbate-2-phosphate of 50µg/ml, TGF-β3 of 10 ng/ml, insulin of 6.25µg/ml, transferrin of 6.25µg/ml, selenic acid of 6.25µg/ml, linoleic acid of 5.33µg/ml, bovine serum albumin of 1.25 mg/ml) at the density of 2×10⁶ cells/cm². 0.5 ml of the transplant cells 2 were supplied and seeded in the scaffold 7. The scaffold 7 together with the culture medium was entered into a centrifugal tube containing a differentiation medium of 50ml, and rotated and cultured at 37°C for four weeks, while the differentiation medium was changed once every three days by the same method as that in Example 2. Then, the cells 2 were transferred to a centrifugal tube containing 50 ml of an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM), and rotated and cultured at 37°C for four weeks, while the differentiation medium was changed once every three days by the same method as that in Example 2. When the scaffold 7 according to Example 13 was cut to observe the inside of it, the transplant cells 2 were seeded in an approximately whole small hole structure of the scaffold 7.

### <Example 14>

Transplant cells 1 were suspended with an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM) at the density of 2×10⁶ cells/cm². 0.5 ml of the transplant cells 1 were supplied and seeded in the scaffold 8. The scaffold 8 together with the differentiation medium was entered into a centrifugal tube containing a differentiation medium of 50ml, and rotated and cultured at 37°C for one week, while the differentiation medium was changed once every three days by the same method as that in Example 2. When the scaffold 8 according to Example 14 was cut to observe the inside of it, the transplant cells 1 were seeded in an approximately whole small hole structure of the scaffold 7.

### <Example 15>

Transplant cells 1 were suspended with a chondrogenic differentiation medium (αMEM, glucose of 4.5 mg/ml, 10⁻⁷M dexamethasone, ascorbate-2-phosphate of 50µg/ml, TGF-β1 of 10 ng/ml, insulin of 6.25µg/ml, transferrin of 6.25µg/ml, selenic acid of 6.25µg/ml, linoleic acid of 5.33µg/ml, bovine serum albumin of 1.25 mg/ml) at the density of 2×10⁶ cells/cm². 0.5 ml of the transplant cells 1 were supplied and seeded in the scaffold 9. The scaffold 9 together with the differentiation medium was entered into a centrifugal tube containing a differentiation medium of 50ml, and rotated and cultured at 37°C for four weeks, while the differentiation medium was changed once every three days by the same method as that in Example 2. When the scaffold 9 according to Example 15 was cut to observe the inside of it, the transplant cells 1 were seeded in an approximately whole small hole structure of the scaffold 9.

### <Comparative example 1>

The scaffold 1 was previously dipped in a culture medium (αMEM), and taken into a sterilized desiccator. The pressure in the desiccator was reduced by a vacuum pump so as to make a reduced pressure state, and the culture medium (αMEM) was infiltrated into the inside of the scaffold 1. Then, an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM) was filled in a cell culturing plate where the scaffold 1 is placed. 0.5 ml of the transplant cells 1 suspended with the osteogenic differentiation medium at the density of 2×10⁶ cells/cm² was supplied from the upside and seeded, and kept to stand for two hours. Thereafter, the scaffold 1 together with the differentiation medium were transferred to a centrifugal tube containing a differentiation medium of 50ml, and cultured for two days at 37°C. When the scaffold 1 was cut to observe the inside of it, the transplant cells 1 colored with a succinic acid dehydrogenase were observed as a thin layer having a dark color at the upper part of the scaffold 1. The reason of this result is that the transplant cells 1 were not infiltrated into the inside of the scaffold 1 and concentrated at the upper part of the scaffold.

### <Comparative example 2>

The scaffold 1 was previously dipped in a culture medium (DMEM), and taken into a sterilized desiccator. The pressure in the desiccator was reduced by a vacuum pump so as to make a reduced pressure state, and the culture medium (DMEM) was infiltrated into the inside of the scaffold 1. Then, an osteogenic differentiation medium (αMEM, glucose of 1.0 mg/ml, 10% FBS, 10⁻⁷M dexamethasone, ascorbate-2-phosphate of 50µg/ml, β-glycerophosphate of 10mM) was filled in a cell culturing plate where the scaffold 1 is placed. 0.5 ml of the transplant cells 1 suspended with the osteogenic differentiation medium at the density of 2×10⁶ cells/cm² was supplied from the upside and seeded. Each cell culturing plate was subjected to centrifugal separation for 500 gravity/min. × 5 minutes for trying to forcibly enter the cells into the scaffold 1, and kept to stand for two hours. Thereafter, the scaffold 1 together with the differentiation medium were transferred to a centrifugal tube containing a differentiation medium of 50 ml, and cultured for two days at 37°C. As for the scaffold 1 according to comparative example 2, the transplant cells 1 were not infiltrated into the inside of the scaffold 1, and the transplant cells 1 were concentrated at the upper part of the scaffold 1, like the scaffold 1 according to comparative example 1.

## Claims

1. A production method of a porous cell scaffold comprising steps of:
filling a guiding solution in a whole continuous small hole structure of a sheet-shaped or block-shaped scaffold having a continuous small hole structure with hole diameters of 5 to 3200 µm and an average hole diameter of 50 to 1500 µm, and having a thickness of 2 mm or more;
supplying thereafter a culture medium with cells being suspended to an upper side of the scaffold;
sucking the guiding solution from a lower side of the scaffold by means of a water absorber contacting the lower side of the scaffold; and
entering thereby the culture medium with cells being suspended into the whole small hole structure of the scaffold
wherein the water absorber is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing polymer materials.

2. The method as claimed in claim 1,
wherein kinematic viscosity at a time of use of the guiding solution is 50 to 450 % of kinematic viscosity at a time of use of a culture medium.

3. The method as claimed in claim 1 or 2,
wherein the guiding solution is water, physiological saline, a buffer solution, body fluid, or a culture medium.

4. The method as claimed in claim 1, further comprising steps of:
supplying a new culture medium from an upside to the porous cell scaffold produced by the foregoing steps, where the culture medium with cells being suspended is entered, at every predetermined period of time passing;
removing the culture medium from a lower side of the scaffold by means of a water absorber contacting the lower side of the scaffold so as to change the culture medium;
repeating such change of the culture medium; and
producing thereby a porous cell scaffold containing cultured cells,
wherein the water absorber contacting the lower side of the scaffold is selected from various papers such as a filter paper, a paper towel, a blotting paper, a processed paper, various fibers such as cotton, silica wool, silk, wool, glass wool, rayon, hemp, cellulose acetate, cellulose nitrate, various porous adsorbents such as silica gel, diatomite, cellulose powder, and one or a combination of more kinds of water-absorbing polymer materials.

## Patentansprüche

1. Verfahren zur Herstellung eines porösen Zellengerüsts, umfassend die Schritte:
Füllen einer Führungslösung in die Gesamtheit einer kontinuierlichen Struktur mit kleinen Löchern eines blattförmigen oder blockförmigen Gerüsts, das eine kontinuierliche Struktur mit kleinen Löchern mit Lochdurchmessern von 5 bis 3200 µm und einem durchschnittlichen Lochdurchmesser von 50 bis 1500 µm aufweist, und das eine Dicke von 2 mm oder mehr aufweist,
anschließend Zuführen eines Kulturmediums mit suspendierten Zellen zu einer Oberseite des Gerüsts,
Saugen der Führungslösung von einer unteren Seite des Gerüsts mittels eines Wasserabsorbers, der mit der unteren Seite des Gerüsts in Kontakt ist, und
**dadurch** Einbringen des Kulturmediums mit suspendierten Zellen in die Gesamtheit der Struktur mit kleinen Löchern des Gerüsts,
wobei der Wasserabsorber aus verschiedenen Papieren, wie z.B. einem Filtrierpapier, einem Papierhandtuch, einem Blotting-Papier, einem verarbeiteten Papier, verschiedenen Fasern, wie z.B. Baumwolle, Silicawolle, Seide, Wolle, Glaswolle, Reyon, Hanf, Celluloseacetat, Cellulosenitrat, verschiedenen porösen Adsorptionsmitteln, wie z.B. Silicagel, Diatomit, Cellulosepulver, und einem wasserabsorbierenden Polymermaterial oder einer Kombination von mehreren Arten von wasserabsorbierenden Polymermaterialien ausgewählt ist.

2. Verfahren nach Anspruch 1, bei dem die kinematische Viskosität der Führungslösung zum Zeitpunkt der Verwendung 50 bis 450 % der kinematischen Viskosität eines Kulturmediums zum Zeitpunkt der Verwendung beträgt.

3. Verfahren nach Anspruch 1 oder 2, bei dem die Führungslösung Wasser, physiologische Kochsalzlösung, eine Pufferlösung, eine Körperflüssigkeit oder ein Kulturmedium ist.

4. Verfahren nach Anspruch 1, das ferner die Schritte umfasst:
Zuführen eines neuen Kulturmediums von einer Oberseite zu dem porösen Zellengerüst, das durch die vorstehend genannten Schritte erzeugt worden ist, wobei das Kulturmedium mit suspendierten Zellen nach jedem vorbestimmten vergangenen Zeitraum eingebracht wird,
Entfernen des Kulturmediums von einer unteren Seite des Gerüsts mittels eines Wasserabsorbers, der mit der unteren Seite des Gerüsts in Kontakt ist, so dass das Kulturmedium ausgetauscht wird,
Wiederholen eines solchen Austauschs des Kulturmediums und
**dadurch** Erzeugen eines porösen Zellengerüsts, das kultivierte Zellen enthält,
wobei der Wasserabsorber, der mit der unteren Seite des Gerüsts in Kontakt ist, aus verschiedenen Papieren, wie z.B. einem Filtrierpapier, einem Papierhandtuch, einem Blotting-Papier, einem verarbeiteten Papier, verschiedenen Fasern, wie z.B. Baumwolle, Silicawolle, Seide, Wolle, Glaswolle, Reyon, Hanf, Celluloseacetat, Cellulosenitrat, verschiedenen porösen Adsorptionsmitteln, wie z.B. Silicagel, Diatomit, Cellulosepulver, und einem wasserabsorbierenden Polymermaterial oder einer Kombination von mehreren Arten von wasserabsorbierenden Polymermaterialien ausgewählt ist.

## Revendications

1. Procédé de production d'une charpente cellulaire poreuse, comprenant les étapes consistant à :
- remplir d'une solution de guidage, toute une structure à petits trous continus d'une charpente en forme de feuille ou en forme de bloc, ayant une structure à petits trous continus avec des diamètres de trous allant de 5 à 3200 µm et un diamètre moyen de trou allant de 50 à 1500 µm, et une épaisseur de 2 mm ou plus ;
- introduire ensuite un milieu de culture contenant des cellules en suspension par une face supérieure de la charpente ;
- aspirer la solution de guidage par une face inférieure de la charpente à l'aide d'un milieu absorbant l'eau en contact avec la face inférieure de la charpente, et
- faire entrer de cette manière, le milieu de culture avec des cellules en suspension dans toute la structure à petits trous de la charpente,
où le milieu absorbant l'eau est choisi parmi différents papiers comme un papier filtre, une serviette en papier, un papier de transfert, un papier traité, différentes fibres telles que le coton, la laine de silice, la soie, la laine, la laine de verre, le rayonne, le chanvre, l'acétate de cellulose, le nitrate de cellulose, différents adsorbants poreux tels que le gel de silice, la diatomite, la poudre de cellulose, et un ou une combinaison de plusieurs types de matériaux polymères absorbant l'eau,

2. Procédé selon la revendication 1, où la viscosité cinétique au moment de l'utilisation de la solution de guidage se situe dans l'intervalle allant de 50 à 450% de la viscosité cinématique au moment de l'utilisation d'un milieu de culture.

3. Procédé selon la revendication 1 ou 2, où la solution de guidage est l'eau, le sérum physiologique, une solution tampon, un fluide corporel ou un milieu de culture.

4. Procédé selon la revendication 1, comprenant en outre, les étapes consistant à :
- introduire un nouveau milieu de culture depuis une face supérieure de la charpente cellulaire poreuse produite dans les étapes précédentes, où le milieu de culture avec les cellules en suspension est introduit à chaque écoulement d'une période prédéterminée ;
- enlever le milieu de culture par une face inférieure de la charpente à l'aide d'un milieu absorbant l'eau en contact avec la face inférieure de la charpente de manière à changer le milieu de culture ;
- répéter un tel changement de milieu de culture, et
- produire ainsi une charpente cellulaire poreuse contenant des cellules en culture,
où le milieu absorbant l'eau entrant en contact avec la face inférieure de la charpente est choisi parmi différents papiers comme un papier filtre, une serviette en papier, un papier de transfert, un papier traité, différentes fibres telles que le coton, la laine de silice, la soie, la laine, la laine de verre, le rayonne, le chanvre, l'acétate de cellulose, le nitrate de cellulose, différents adsorbants poreux tels que le gel de silice, la diatomite, la poudre de cellulose, et un ou une combinaison de plusieurs types de matériaux polymères absorbant l'eau.
